(19) Europäisches Patentamt
European Patent Office
Office européen des brevets

(11) **EP 4 518 155 A1**

(12) **EUROPEAN PATENT APPLICATION**

(43) Date of publication:
**05.03.2025 Bulletin 2025/10**

(21) Application number: **23193810.1**

(22) Date of filing: **29.08.2023**

(51) International Patent Classification (IPC):
*H03F 3/45* (2006.01)   *A61B 5/305* (2021.01)

(52) Cooperative Patent Classification (CPC):
**H03F 3/45246; A61B 5/305; H03F 3/45668;**
**H03F 3/45677;** H03F 2200/261; H03F 2203/45406;
H03F 2203/45441

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Imec VZW**
**3001 Leuven (BE)**

(72) Inventors:
• **Sawigun, Chutham**
**3001 Leuven (BE)**

• **Mora Lopez, Carolina**
**3001 Heverlee (BE)**
• **Yang, Xiaolin**
**3000 Leuven (BE)**
• **Aymerich, Joan**
**3012 Leuven (BE)**

(74) Representative: **Roth, Sebastian**
**Mitscherlich PartmbB**
**Patent- und Rechtsanwälte**
**Karlstraße 7**
**80333 München (DE)**

(54) **COMMON MODE INTERFERENCE SUPPRESSION IN AN AMPLIFIER CIRCUIT FOR A NEUROMODULATION DEVICE**

(57)    This disclosure provides a neuromodulation device for measuring a biological electrical signal in response to a neural modulation of biological tissue. The neuromodulation device comprises at least one amplifier circuit (20), which is used to amplify an input voltage signal that is based on the biological electrical signal. The amplifier circuit (20) suppresses a common mode (CM) voltage signal in the input voltage signal. The amplifier circuit (20) comprises an input stage (21) to receive the input voltage signal, and a differential trans-conductor (23) to provide an output current signal based on a DM voltage signal in the input voltage signal. The transconductor (23) provides a first CM voltage signal and a second CM voltage signal to a CM amplifier (27) of the amplifier circuit (20). The CM amplifier (27) combines the first CM voltage signal with the second CM voltage signal with an inverting gain, and provides the inverted CM voltage signal back to the transconductor (23) for enabling the CM suppression.

**FIG. 2**

EP 4 518 155 A1

**Description**

TECHNICAL FIELD

The present disclosure provides a neuromodulation device for measuring a biological electrical signal in response to a neural modulation of biological tissue. The neuromodulation device comprises at least one amplifier circuit, which is used to amplify an input voltage signal that is based on the biological electrical signal. The amplifier circuit provided by this disclosure is configured to suppress a common mode (CM) voltage signal in the input voltage signal, and consequently to reduce CM interference (CMI).

BACKGROUND

A bi-directional (i.e., closed loop) neural interface may be used by a device to perform both neural stimulation and recording. Such a device enables neuromodulation - i.e., is referred to as a neuromodulation device - which is useful for neuroscience studies and treatments of neurological disorders.

For performing the neural recording, an amplifier circuit (also referred to as neural amplifier) is required, and should be able to perform low-noise small-signal amplification, while at the same time rejecting a large electrode DC offset (EDO). To achieve the desired EDO rejection ability, capacitively coupled architectures have been widely adopted for such amplifier circuits, since the input capacitors exhibit infinite impedance at DC, and thus prevent the EDO from being amplified. To achieve the low-noise performance, a differential inverter-based transconductor is advantageously used in the amplifier circuit, since it provides a higher transconductance than other well-known types of transconductor (e.g., simple wide-swing, or folded cascode).

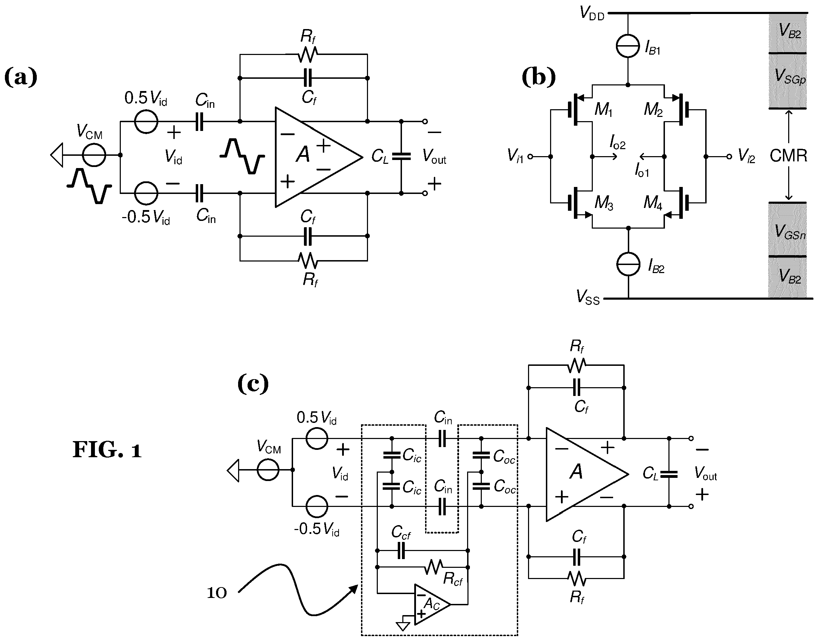

An example of a conventional capacitively coupled amplifier circuit that may be used in a neuromodulation device is shown in FIG. 1(a). The amplifier circuit has an inverter as input stage, which is shown in FIG. 1(b). When recording in a controlled environment, the amplifier circuit of FIG. 1(a) performs reasonably well, since there is no severe CMI disturbing its performance. However, for a bi-directional neural interface, the amplifier circuit may suffer from CMI due to its limited CM range (CMR).

In particular, during performing the neural stimulation, depending on the distance between the place of stimulation to recording electrodes, and on how strong the stimulation signal is, the stimulation signal can partially propagate to the input (recording and reference electrodes) of the amplifier circuit of FIG. 1(a), as a stimulation waveform-like CM voltage ($V_{CM}$, also referred to as CM voltage signal). A high-pass cut-off frequency $f_h$ in the amplifier circuit is defined by the resistor $R_f$ and the capacitor $C_f$, namely as $f_h = 1/(2\pi C_f R_f)$, and is typically set below 1 Hz. The $V_{CM}$ can travel to appear at the input of the amplifier circuit, if the stimulation frequency is higher than $f_h$ (and it mostly is). Considering the amplifier's input stage shown in FIG. 1(b), and due to its stacking nature, the CMR of the amplifier is limited to $V_{DD}$-$V_{SS}$-($V_{B1}+V_{B2}+VGS_n+VSG_p$), where $V_{B1}$ and $V_{B2}$ are minimum voltage drops required by tail current sources $I_{B1}$ and $I_{B2}$, respectively. The other parameters have their own usual meanings. The $V_{CM}$ can be in the range of 50-300 mV or even greater. For these reasons, the input stage of FIG. 1(b) cannot always handle the $V_{CM}$, and consequently the amplification of the amplifier circuit of FIG. 1(a) may fail.

To address this problem, a CM feedforward (CMFF) amplifier may be inserted into the amplifier circuit, as shown in FIG. 1(c). The CMFF amplifier contains a transconductor (A), a pair of CM detector capacitors $C_{ic}$, a feedback capacitor $C_{cf}$, a resistor $R_{cf}$, and current stealing capacitors $C_{oc}$. This amplifier circuit detects and amplifies the $V_{CM}$ with an AC gain of -$2C_{ic}/C_{cf}$. The input CM current flowing through $C_{in}$ (i.e., $I_{CM}$) can be calculated by setting the CM input terminal of the amplifier circuit to ground (i.e., $V_i+ = V_i- = 0$). The CMFF can then be designed to steal $I_{CM}$ away from that node through capacitors $C_{oc}$ under the condition that $C_{oc} = C_{in}C_{cf}/(2C_{ic})$. By doing so, the input CM voltage fluctuations at the input terminal of the amplifier circuit can be minimized. The inverter based input stage can remain functioning for $V_{CMpp}$ > CMR.

However, the additional CMFF amplifier shown in FIG. 1(c) adds more input capacitor $C_{ic}$ in addition to the original $C_{in}$, thereby degrading the input impedance of the amplifier circuit. In case $C_{ic} = C_{in}$ is set, the input impedance may be halved. This resulting smaller input impedance is not desirable, since it attenuates the signal and enlarges the input-referred noise (IRN). To compensate for this impedance degradation, the amplifier circuit of FIG. 1(c) may further incorporate an impedance boosting network, which leads to additional hardware complexity. However, especially for multichannel closed-loop neural interfaces, the chip area per channel needs to be kept tiny, so as to accommodate several hundreds of channels or more within a reasonable total chip area. The large chip area per channel as required by the design of FIG. 1(c) may thus not be acceptable.

SUMMARY

In view of the above, this disclosure aims to provide an improved amplifier circuit for a neuromodulation device, in particular, to overcome the above-mentioned issues. An objective is accordingly to suppress a CM voltage signal and reduce CMI, while keeping the size of the amplifier circuit small. A further objective is to avoid input impedance degradation. An objective is also the performance of the amplifier circuit for low-noise small-signal amplification, and that the amplifier circuit at the same time rejects large EDO. Another objective of this disclosure is to provide an improved neuromodulation device.

These and other objectives are achieved by the solutions provided in the independent claims. Advantageous implementations are described in the dependent claims.

A first aspect of this disclosure provides an amplifier circuit for amplifying a biological electrical signal, the amplifier circuit comprising: an input stage with two input terminals, configured to receive an input voltage signal, which is based on the biological electrical signal and comprises a CM voltage signal and a differential mode (DM) voltage signal; a differential transconductor comprising a non-inverting input and an inverting input, each connected to one of the two input terminals, and comprising an output for providing an output current signal based on the DM voltage signal; wherein the transconductor is configured to provide a first CM voltage signal, which is tapped after the non-inverting input, and a second CM voltage signal, which is tapped after the inverting input; and a CM amplifier configured to combine the first CM voltage signal with the second CM voltage signal to obtain a combined CM voltage signal, amplify the combined CM voltage signal with an inverting gain to obtain an inverted CM voltage signal, and provide the inverted CM voltage signal to the non-inverting input and the inverting input of the transconductor.

The CM amplifier of the amplifier circuit of the first aspect suppresses the CM voltage signal in the input signal and reduces CMI. Since the amplifier circuit comprises the CM amplifier after the transconductor, input impedance degradation is avoided. Also, the size of the amplifier circuit can be smaller than, for example, the amplifier circuit shown in FIG. 1(c). Due to the differential transconductor, a good low-noise performance of the amplifier circuit can be achieved.

In an implementation, the amplifier circuit comprises: a first capacitive feedback line configured to feedback the inverted CM voltage signal from the CM amplifier to the non-inverting input of the transconductor; and a second capacitive feedback line configured to feedback the inverted CM voltage signal from the CM amplifier to the inverting input of the transconductor; wherein each of the first and the second capacitive feedback line comprises a first capacitor.

In an implementation of the amplifier circuit, each of the non-inverting input and the inverting input of the transconductor is connected to one of the two input terminals of the input stage via a respective second capacitor; and the second capacitor has a higher capacitance than the first capacitor.

The capacitive feedback from the CM amplifier according to the above implementations leads to low noise in the amplifier circuit.

In an implementation of the amplifier circuit, the transconductor comprises: a first transistor connected with its gate to the non-inverting input and coupled with its source to a supply voltage, VDD; a second transistor connected with its gate to the inverting input, connected with its source to the source of the first transistor, and coupled with its source to VDD; a third transistor connected with its gate to the non-inverting input, connected with its drain to the drain of the first transistor, and coupled with its source to a ground voltage; and a fourth transistor connected with its gate to the inverting input, connected with its drain to the drain of the second transistor, connected with its source to the source of the third transistor, and coupled with its source to VSS; wherein the first CM voltage signal is tapped between the sources of the first and the second transistor, and the second CM voltage signal is tapped between the sources of the third and the fourth transistor.

In an implementation, the amplifier circuit is configured such that a tail current of the source-connected third and fourth transistor is higher than a tail current of the source-connected first and second transistor in use of the amplifier circuit.

This allows folded-cascode branches to deliver the output current signal of the amplifier circuit.

In an implementation of the amplifier circuit, the CM amplifier comprises a summing junction at which the first CM voltage signal and the second CM voltage signal are combined into the combined CM voltage signal; wherein each of the first CM voltage signal and the second CM voltage signal is connected to the combination point via a respective third capacitor.

In an implementation of the amplifier circuit, the CM amplifier further comprises a gain circuit including a second transconductor, a fourth capacitor and a first resistor; and the gain circuit is configured to amplify the combined CM voltage signal with the inverting gain to produce the inverted CM voltage signal.

In an implementation, the amplifier circuit further comprises a DM feedback circuit configured to provide a feedback voltage signal, which is based on the output current signal of the transconductor, to the non-inverting input and the inverting input of the transconductor.

In an implementation of the amplifier circuit, the DM feedback circuit comprises a first capacitive feedback path and a second capacitive feedback path; and each capacitive feedback path comprises a respective fifth capacitor that is connected in parallel to a respective second resistor.

The capacitively coupled feedback architecture of the above implementations achieves the desired EDO rejecting abilities of the amplifier circuit.

In an implementation, the amplifier circuit further comprises a conversion circuit configured to convert the output current signal of the transconductor into the feedback voltage signal.

In an implementation of the amplifier circuit, the biological electrical signal is an electroencephalogram, (EEG) signal or a stimulation-based neural signal.

A second aspect of this disclosure provides a neuromodulation device for measuring one or more biological electrical signals in response to a neural modulation of biological tissue, wherein the neuromodulation device comprises one or more amplifier circuits of the first aspect or any implementation thereof.

In an implementation, the neuromodulation device further comprises an array of electrodes comprising a first set of electrodes and a second set of electrodes, wherein each electrode of the first set is connected to a respective amplifier circuit of the one or more amplifier circuits, and wherein each electrode of the second set is configured to cause a respective neural modulation of the biological tissue.

That is, some electrodes can be used for recording, and some other electrodes for stimulating, which may be beneficial for the performance of the neuromodulation device.

In an implementation of the neuromodulation device, each electrode of the second set comprises a stimulator, which is configured to provide a stimulation signal to cause the neural modulation of the biological tissue.

In an implementation, the neuromodulation device comprises multiple of the amplifier circuits; wherein the amplifier circuits share a single CM amplifier; and the CM amplifier is configured to provide, for each of the amplifier circuits, a respective inverted CM voltage signal to the non-inverting input and the inverting input of the transconductor of that amplifier circuit.

This allows reducing the size of the neuromodulation device significantly, in particularly, reducing the area per channel.

In an implementation, the neuromodulation device further comprises one or more shared buffers connected to the multiple amplifier circuits and to the single CM amplifier, wherein the output of each amplifier circuit is connected by a respective switch to the shared buffers; wherein the neuromodulation device is configured to sequentially operate the switches, which causes the multiple amplifier circuits to sequentially output their respective current signal and causes the CM amplifier to sequentially provide the respective inverted CM voltage signal to the respective transconductor of one of the amplifier circuits.

The shared buffers allow multiplexing the multiple channels.

In an implementation, the neuromodulation device further comprises a bi-directional neural interface comprising at least one needle; wherein the neural interface is configured to provide the neural stimulation to the biological tissue, to receive the one or more biological electrical signals, and to provide them to the one or more amplifier circuits.

The neuromodulation device of the second aspect achieves the same advantages as the amplifier circuit of the first aspect.

BRIEF DESCRIPTION OF THE DRAWINGS

The above described aspects and implementations are explained in the following description of embodiments with respect to the enclosed drawings:

FIG. 1      shows exemplary amplifier circuits for a neuromodulation device, in particular, (a) without CM suppression and (b) with CM suppression circuitry.

FIG. 2      shows a general architecture of an amplifier circuit for a neuromodulation device according to this disclosure.

FIG. 3      shows an exemplary amplifier circuit for a neuromodulation device according to this disclosure.

FIG. 4      shows the transistor-level of the amplifier circuit of FIG. 3.

FIG. 5      compares output voltage signals of an exemplary amplifier circuits having (a) no CM suppression and (b) a CM amplifier for CM suppression according to this disclosure.

FIG. 6      shows an exemplary neuromodulation device according to this disclosure.

FIG. 7      shows an example of a neuromodulation device according to this disclosure, which comprises an array of electrodes and a needle as bidirectional neural interface.

FIG. 8      shows a multichannel recording arrangement according to this disclosure with a shared CM amplifier and shared buffers.

FIG. 9      shows a simulated channel discrete Fourier transform (DFT) of a 16-channel architecture of the multichannel recording arrangement according to this disclosure, in particular, (a) without reset switches and (b) with reset switches.

DETAILED DESCRIPTION OF EMBODIMENTS

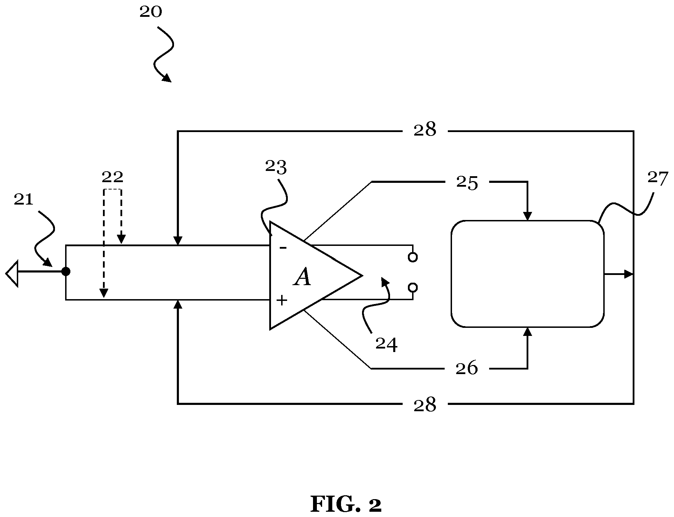

FIG. 2 shows an amplifier circuit 20 according to this disclosure. The amplifier circuit 20 is configured to amplify a biological electrical signal, for instance, as received in response to a neural modulation of biological tissue. That is, the biological electrical signal maybe a stimulation-based neural signal. The biological electrical signal may, for example, be an EEG signal. The amplifier circuit 20 is suitable for use in a neuromodulation device or a recording arrangement. Due to its low-noise performance and efficient CM suppression, the amplifier circuit 20 is especially suited for a neuromodulation device using a bi-directional neural interface for neural stimulation and signal recording.

The amplifier circuit 20 shown in FIG. 2 comprises an input stage 21 with two input terminals, a differential transconductor 23, and a CM amplifier 27.

The input stage 21 is configured to receive an input voltage signal 22, which is based on the biological electrical signal that the amplifier circuit 20 is intended to amplify. For instance, the input voltage signal 22 may be derived from the biological electrical signal, or may be the biological electrical signal, or the biological electrical signal may be signal processed to obtain the input voltage signal 22. The input voltage signal 22 comprises a CM voltage signal and a DM voltage signal. The DM voltage signal is the signal component, which the amplifier circuit 20 is supposed to amplify, while the CM voltage signal is the signal component that the amplifier circuit 20 is designed to suppress.

The differential transconductor 23 comprises a non-inverting input and an inverting input, like a differential amplifier. Each input of the transconductor 23 is connected to one of the two input terminals, i.e., the input voltage signal 22 is provided to the differential transconductor 23. The transconductor 23 further comprises an output 24, which is configured to provide an output current signal of the amplifier circuit 20, wherein the output current signal is based on the DM voltage signal. For instance, the output current signal may amplify the DM voltage signal. The amplifier circuit 20 is further configured to suppress the CM voltage signal in the input voltage signal 22 from appearing or at least substantially influencing the output current signal.

To this end, the transconductor 23 is configured to provide a first CM voltage signal 25 and a second CM voltage signal 26 to the CM amplifier 27. The first CM voltage signal is tapped after the non-inverting input, for instance, between the non-inverting input and the output 24 of the transconductor 23. The second CM voltage signal 26 is tapped after the inverting input, for instance, between the inverting input and the output 24 of the transconductor 23. Exemplary details where the CM voltage signals 25, 26 are tapped are provided below.

The CM amplifier 27 is configured to combine the first CM voltage signal 25 with the second CM voltage signal 26, so as to obtain a combined CM voltage signal. For instance, the first and second CM voltage signal 25, 26 may be summed. Then, the CM amplifier 27 is configured to amplify the combined CM voltage signal with an inverting gain, so as to obtain an inverted CM voltage signal 28. This inverted CM voltage signal 28 is then provided back to the non-inverting input and the inverting input of the transconductor 23, respectively, as indicated in FIG. 2. This feedback leads to a suppression of the CM voltage signal in the output current signal of the transconductor 23.

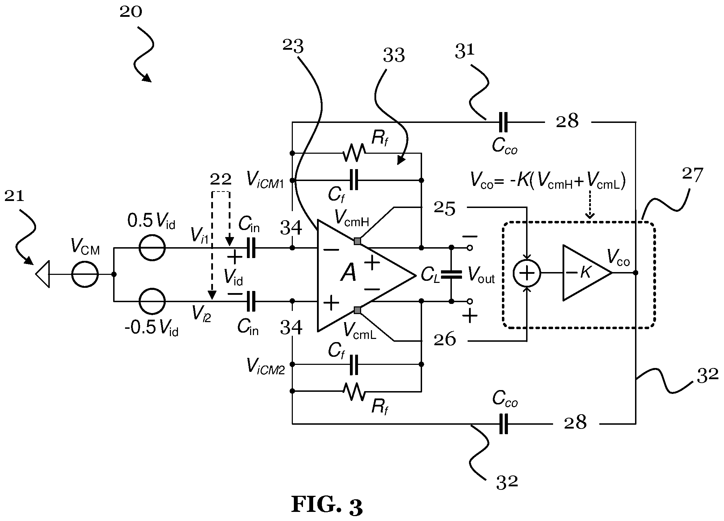

FIG. 3 shows an exemplary and more detailed amplifier circuit 20 according to this disclosure, which builds on the amplifier circuit 20 shown in FIG. 2. Same components in FIG. 2 and FIG. 3 share the same reference signs and function likewise.

The amplifier circuit 20 shown in FIG. 3 comprises capacitive feedback lines to provide the inverted CM voltage signal 28 back to the inputs of the transconductor 23. The feedback lines of the amplifier circuit include a first capacitive feedback line 31, which is configured to feedback the inverted CM voltage signal 28 from the CM amplifier 27 to the non-inverting input of the transconductor 23, and a second capacitive feedback line 32, which is configured to feedback the inverted CM voltage signal 28 from the CM amplifier 27 to the inverting input of the transconductor 23. Both capacitive feedback lines 31 and 32 comprise a capacitor $C_{co}$.

The amplifier circuit 20 comprises further capacitors. For instance, the amplifier circuit 20 comprises a capacitor $C_L$ at the output 24, in particular, between two output terminals of the output 24. Further, the amplifier circuit 20 comprises two capacitors $C_{in}$. Namely, both the non-inverting input and the inverting input of the transconductor 23 may be connected via one capacitor $C_{in}$ to the input stage 21. The capacitors $C_{in}$ are preferably selected such that they have (each) a higher capacitance than the capacitors $C_{co}$.

The amplifier circuit 20 further comprises a DM feedback circuit 33, which is configured to provide a feedback voltage signal 34, which is based on the output current signal of the transconductor 23 - which is again based on the DM voltage signal in the input voltage signal 22 - to the two inputs of the transconductor 23. The DM feedback circuit 33 comprises a first and a second capacitive feedback path, wherein each feedback path comprises a capacitor $C_f$ and a resistor $R_f$ that is connected in parallel to $C_f$.

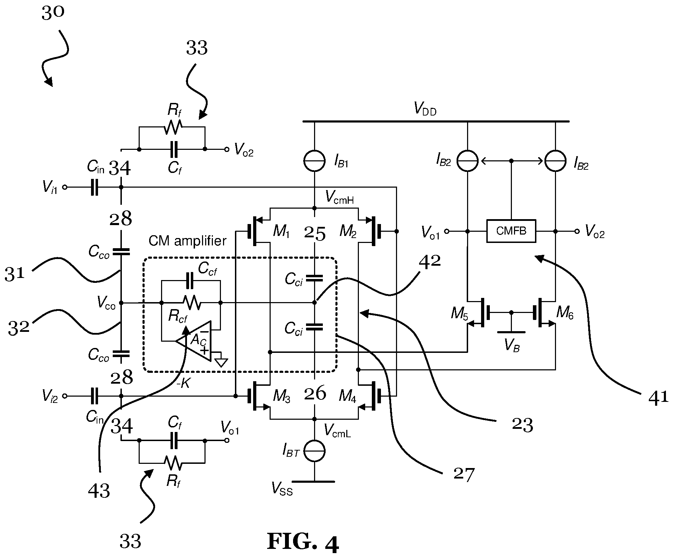

FIG. 4 shows an exemplary transistor-level implementation of an amplifier circuit 20 according to this disclosure, for example, the amplifier circuit 20 shown in FIG. 3. Same components in FIG. 3 and FIG. 4 share the same reference signs and function likewise.

As can be seen in FIG. 4, the transconductor 23 comprises four transistors $M_1$, $M_2$, $M_3$, and $M_4$. The first transistor $M_1$ is connected with its source to the source of the second transistor $M_2$. Similarly, the third transistor $M_3$ is connected with its source to the source of the fourth transistor $M_4$. Accordingly, the pair $M_1$-$M_2$ and the pair $M_3$-$M_4$ is, respectively, a source-connected transistor pair. These two transistor pairs are connected to each other. In particular, the first transistor $M_1$ is connected with its drain to the drain of the third transistor $M_3$, and similarly the second transistor $M_2$ is connected with its drain to the drain of the fourth transistor $M_4$.

Further, the gates of the first transistor $M_1$ and the third transistor $M_3$ are both connected to the non-inverting input, and the gates of the second transistor $M_2$ and the fourth transistor $M_4$ are both connected to the inverting input. Moreover, the connected sources of the first transistor $M_1$ and the second transistor $M_2$ are further coupled to $V_{DD}$, e.g., via a current source, and the connected sources of the third transistor $M_3$ and the fourth transistor $M_4$ are further coupled to Vss, e.g., via a current source. Notably, in this disclosure "connected" typically denotes a direct electrical connection between two components, while "coupled" denotes and indirect connection, for instance, via one or more other components.

In the exemplary amplifier circuit 20 of FIG. 4, the first CM voltage signal 25 is tapped between the sources of the first and the second transistor $M_1$, $M_2$, and the second CM voltage signal 26 is tapped between the sources of the third and the fourth transistor $M_3$, $M_4$, for instance, at the respective coupling connection of these sources to either $V_{DD}$ and Vss.

These CM voltage signals 25, 26 are provided to the CM amplifier 27, in particular, are routed to a summing junction 42. The lines carrying the first CM voltage signal 25 and the second CM voltage signal 26 may be respectively connected to the summing junction 42 via a respective capacitor $C_{ci}$. At the summing junction 42, the first CM voltage signal 25 and the second CM voltage signal 26 are combined, i.e., added together. This results in the combined CM voltage signal. The combined CM voltage signal is further provided to a gain circuit 43 of the CM amplifier 27. The gain circuit 43 includes a transconductor $Ac$, a capacitor $C_{cf}$ and a resistor $R_{cf}$ as shown, and is configured to amplify the combined CM voltage signal with the inverting gain (negative gain), which results in the inverted CM voltage signal 28. The inverted voltage signal 28 is provided via the capacitive feedback lines 31, 32 to the inverting and non-inverting input of the transconductor 23.

An underlying concept of the amplifier circuits 20 shown in the FIGs. 2-4 is that instead of detecting the CM voltage signal at the input stage 21 and performing feedforward cancellation - as shown for the exemplary amplifier circuit of FIG. 1(c) - the CM voltage signal of the input voltage signal 22 is allowed to enter the transconductor 23, and a feedback-based CM suppression is performed. This helps to avoid the undesired input impedance degradation.

The transconductor 23 (also labelled with A in the FIGs. 3 and 4 provides the two CM voltage signals $V_{cmH}$ and $V_{cmL}$. These voltage signals are then combined and amplified with the inverting gain $-K$. The resulting output of the CM amplifier 27 is the inverted CM voltage signal 28 (also denoted $V_{CO}$), which is determined by

$$V_{CO} = -KV_{ci} = -K(V_{cmH} + V_{cmL}) = -KV_{iCM}.$$

In the above formula, $V_{iCM1} = V_{iCM2} \cong 2V_{cmH} \cong 2V_{cmL}$. The inverted CM voltage signal 28 is fed back to the input terminals of the transconductor 23, via the feedback lines 31, 32 including the capacitors $C_{co}$. This feedback mechanism creates a large equivalent capacitance $C_{EQ}$ to ground, as seen by the CM voltage signals which is defined by

$$C_{EQ} = 2(1 + K)C_{CO}.$$

With this large equivalent capacitance $C_{EQ}$, and for $K \gg 1$, the signal $V_{iCM}$ can be calculated by:

$$V_{iCM} = V_{CM}\left(\frac{2C_{in}}{2C_{in}+C_{EQ}}\right) \cong V_{CM}\left(\frac{C_{in}}{C_{in}+KC_{OC}}\right) \qquad (1).$$

For the ideal case that $K = \infty$, $V_{iCM}$ becomes zero, meaning that there are no CM voltage fluctuations at the inputs of the transconductor 23. In practice, it is advantageous that $C_{CO} \ll C_{in}$ for noise reasons. For example, a practical value of $K > 50$ is desirable. For DM operation, $V_{CO}$ maybe seen as AC ground, and the pair of capacitors $C_{CO}$ (without gain) may be virtually grounded.

In the transistor-level implementation shows in FIG. 4, the tail current of source-coupled transistor pair $M_3$-$M_4$ may be set higher than the tail current of the transistor pair $M_1$-$M_2$) as $I_{BT} = I_{B1} + 2I_{B2}$. This is to allow folded-cascode branches ($M_5$-$I_{B2}$ and $M_6$-$I_{B2}$) to deliver the output current of the input stage to the output nodes $V_{o1}$ and $V_{o2}$, respectively. This topology offers a wide signal swing at the output. The output common mode feedback block (CMFB) may be is inserted to create a CM feedback loop to stabilize the output CM level of $V_{o1}$ and $V_{o2}$. $V_B$ is to bias the common gate voltage

of $M_5$ and $M_6$.

Apart from functioning as the differential transconductor 23, the source-coupled transistor pairs $M_1$-$M_2$ and $M_3$-$M_4$ are also able to detect CM voltage fluctuations, and shift them to appear at the common source terminals of the two transistor pairs as $V_{cmH}$ and $V_{cmL}$, respectively. The CM amplifier 27, which may be formed by formed by $C_{ci}$, $C_{cf}$, $R_{cf}$ and the transconductor $Ac$, will perform CM signal amplification with a gain of $-K = -2C_{Ci}/C_{cf}$. As in the traditional way of designing an amplifier circuit for a neuromodulation device, the resistor $R_{cf}$ can be made by a MOS pseudo-resistor. Thus, together with a small $C_{cf}$, a high-pass cut-off frequency below 1 Hz can be achieved within a tiny chip area. Besides, the transconductor $A_C$ only contributes CM noise that can be cancelled out in the differential mode. It can thus be designed in a compact and low-power fashion. Therefore, a large $K$ can be achieved within a reasonable area.

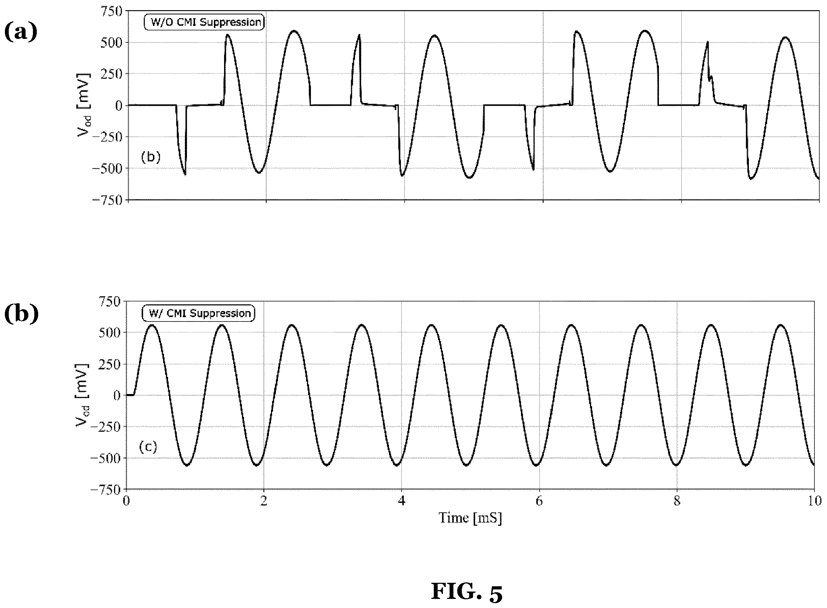

FIG. 5 compares exemplary output voltages of an amplifier circuit without CMI suppression (shown in FIG. 1(a)) and an amplifier circuit 20 comprising the CM amplifier 27 according to this disclosure. The comparison shows that a large CMI will exceed the CMR of the exemplary amplifier circuit, which may shutdown the amplifier circuit as visible in FIG. 5(a). The amplifier circuit 20 of this disclosure is more robust, as the CM is suppressed, and brings back a clean output signal in FIG. 5(b).

In particular, for the results of FIG. 5, a stimulation-like waveform of $V_{CM}$ (CM voltage signal) was applied together with a sinusoidal $V_{id}$ (DM voltage signal) to the amplifier circuits, and the relevant responses are shown in FIG. 5(a) and (b), respectively. FIG. 5(a) shows the output voltage of the exemplary amplifier circuit without the CM amplifier 27 and without capacitive feedback via $C_{CO}$ over time. At the time that the magnitude of $V_{i1}$ is approximately 200 mV, the amplifier circuit fails to provide any output signal. This implies, for instance, that reading a neural signal at a stimulation moment would not be possible. On the other hand, with the help of the CM amplifier 27 and the capacitive feedback via $C_{CO}$, the proper sinusoidal output voltage signal can be obtained, as shown in FIG. 5(b). Recording a neural signal during stimulation is possible in this case, i.e., with the amplifier circuit 20 of this disclosure.

Notably, for the amplifier circuit 20 used in FIG. 5(b), a 500 Hz sinusoidal $V_{CM}$ with a 200 mV$_p$ amplitude was used. The main amplifier consumed 1.5 μA and its parameters were set to $C_{in} = 2.1$ pF, $C_f = 15$ fF. The resistors $R_f$ and $R_{cf}$ were identically made by MOS pseudo-resistors. For the CM amplifier 27, $C_{ci} = 0.6$ pF, $C_{cf} = 15$ fF, $C_{co} = 0.6$ pF, and $Ac$ is formed by a simple wide-swing operational transconductance amplifier (OTA), which consumes 600 nA. $V_{iCM}$ is could be suppressed to 2 mV$_p$ (i.e., 100 times smaller than $V_{CM}$).

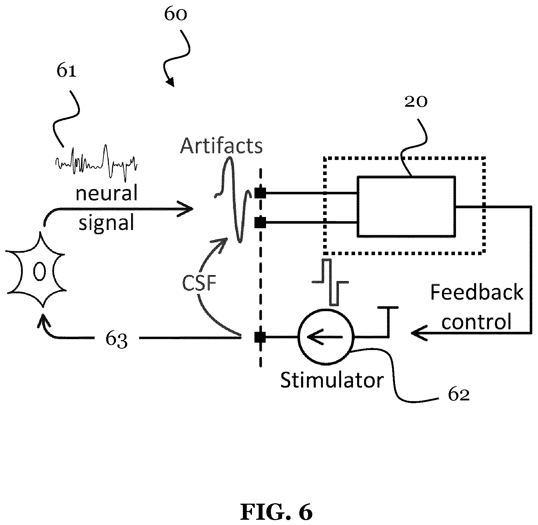

FIG. 6 shows an exemplary neuromodulation device 60 according to this disclosure. The neuromodulation device 60 is configured to measure one or more biological electrical signals 61 in response to a neural modulation of biological tissue. For measuring the one or more biological signals, the neuromodulation device 60 comprises at least one amplifier circuits 20 according to this disclosure, for instance, as described in the FIGs. 2-4.

The neuromodulation device 60 may further comprises at least one stimulator 62, which is configured to generate a stimulation signal 63, which can cause the neural modulation of the biological tissue. The stimulation signal 63 may be provided by a neural interface of the neuromodulation device 60 to the biological tissue. For instance, the neural interface may be a bi-directional neural interface, which is further configured to receive the one or more biological electrical signals 61 in response to the neural stimulation, and to provide them to the one or more amplifier circuits 20.

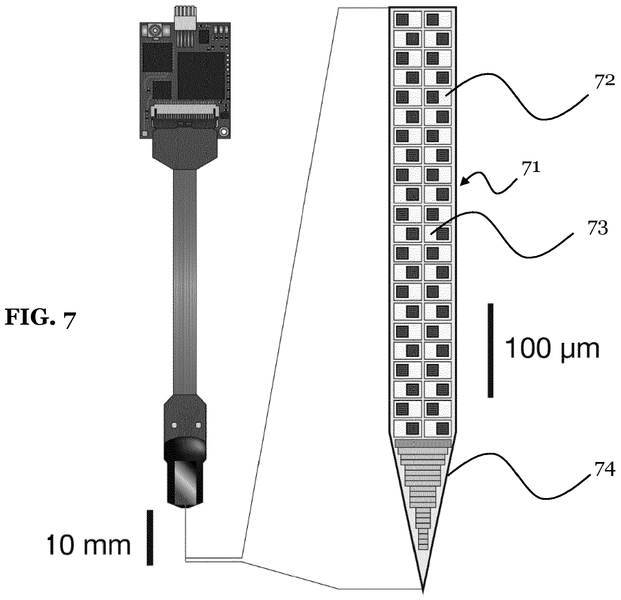

For example, as shown in FIG. 7, the neuromodulation device 60 may comprise a bi-directional neural interface comprising at least one needle 74. Moreover, the neuromodulation device 60 may comprise an array 71 of electrodes. The array 71 may include a first set of electrodes 72 and a second set of electrodes 73, which may be of a different kind or design. In particular, each electrode 72 of the first set may be connected to a respective amplifier circuit 20 of the one or more amplifier circuits 20 of the neuromodulation device 60. Each electrode 73 of the second set may be configured to cause a respective neural modulation of the biological tissue. For instance, each electrode 72 of the first set may comprise at least one amplifier circuit 20, and may be used for measuring (recording) an electrical biological signal 61. Each electrode 73 of the second set may comprises at least one stimulator 62, and may be used to generate a stimulation signal 63 to the interface, in this case comprising the needle 74.

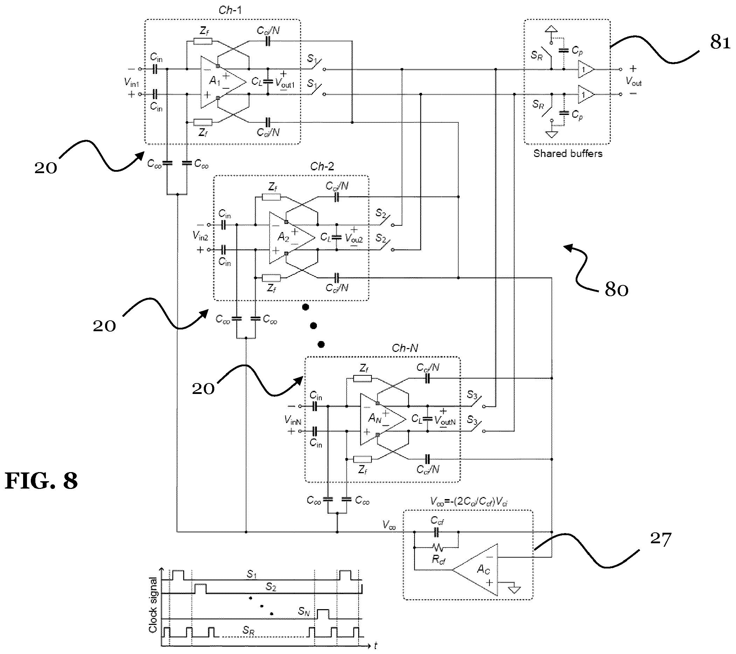

FIG. 8 shows a multichannel recording arrangement 80 according to this disclosure. The recording arrangement 80 comprises multiple of the amplifier circuits 20 according to this disclosure, for example, as shown in FIGs. 2-4. Each amplifier circuit 20 maybe associated with one of multiple channels. The amplifier circuits 20 share a single CM amplifier 27. The CM amplifier 27 may be considered being a part of each amplifier circuit 20. The CM amplifier 27 is configured to provide, for each of the amplifier circuits 20, a respective inverted CM voltage signal 28 to the non-inverting input and the inverting input of the transconductor 23 of that amplifier circuit 20.

The shared CM amplifier 27 may improve the area per channel in the multichannel topology, since it can be shared among many recoding channels. This sharing is possible under the assumption that all recording electrodes (first set of electrodes 72) are placed in the nearby neighborhood and individually suffer from a comparable amount of CMI.

To achieve the same CM feedback gain (i.e., the same degree of CM suppression) for all N channels of the multichannel recording arrangement 80, only the one CM amplifier 27, including one transconductor $Ac$, but including $N$ pairs of $C_{ci}/N$ (the same area occupied by one pair of $C_{ci}$) and a pair of buffers can be used here. A pair of $C_{co}$ is still used for each channel. This topology also offers weighted adjustment for each channel individually by adjusting the $C_{co}$ value. This is useful, if the strength of the CMI appearing on each channel is known beforehand. The CM amplifier 27 may perform summing average of CMI and the required $C_{EQ}$ for each channel, and can be adjusted via the value of $C_{co}$ according to (1) and the known CMI.

The multichannel recording arrangement 80 of FIG. 8 may further comprises one or more shared buffers 81 (which may be used as ADC drivers), which are connected to the multiple amplifier circuits 20 and to the single CM amplifier 27. The output of each amplifier circuit 20 is connected by a respective switch $S_1$, $S_2$, $S_3$...$S_N$ to the shared buffers 81. The outputs of all channels (1 to N) may be multiplexed by switches $S_1$ to $S_N$, and may be controlled by the clock signals shown in timing diagram at the bottom of FIG. 8. That is, the recording arrangement 80 is configured to sequentially operate the switches $S_1$ ... $S_N$, which causes the multiple amplifier circuits 20 to sequentially output their respective current signal, and causes the CM amplifier 27 to sequentially provide the respective inverted CM voltage signal 28 to the respective transconductor 23 of one of the amplifier circuits 20.

After the buffers 81 finish delivering a signal of one channel, further switches $S_R$ (reset switches) may be turned on for a short time, in order to erase memories stored on parasitic capacitances $C_p$, before the buffers 81 receive the signal again from the next consecutive channel. This resetting mechanism helps minimizing channel crosstalk. By sharing the buffers 81, a total power and area consumed and occupied by the buffers 81 will be divided by N when per-channel power and area are calculated.

An embodiment of this disclosure is further a multichannel neuromodulation device, which comprises the multichannel recording arrangement 80 shown in FIG. 8. In addition to the recording arrangement 80, this multichannel neuromodulation device may accordingly comprise at least one least one stimulator, which is configured to generate a stimulation signal. For instance, the multichannel neuromodulation device may be similar to the neuromodulation device 60 of FIG. 6 having multiple channels for recording. For example, the neuromodulation device 60 shown in FIG. 6 may comprise the recording arrangement 80 shown in FIG. 8.

The multichannel scheme of FIG. 8 was evaluated for a 16-channel (N = 16) recording arrangement 80, to verify that the CM amplifier 27 can be shared. A stimulation-like waveform of $V_{CM}$ together with sinusoidal $V_{id}$ (as described above with respect to FIG. 5)) was applied to all channel inputs. Similar results as shown in FIG. 5 were obtained, which proves that the shared CM amplifier 27 is well functional.

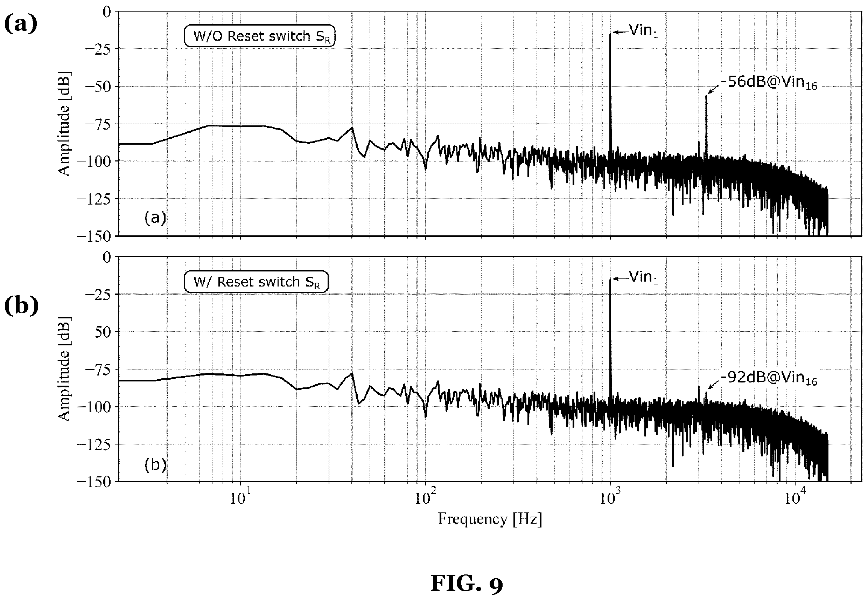

Sharing, for instance, two buffers 81 among the 16 outputs with negligible channel-to-channel crosstalk is also possible thanks to the reset switches $S_R$. FIG. 9 shows a Discrete Fourier Transform (DFT) of a first channel with and without reset mechanism. A 1 kHz 3 mV$_p$ input voltage signal was applied to the first channel. The rest of the channel inputs received the same signal amplitude, but different frequencies that are not set to be clearly visible in frequency domain (not located at any harmonics of the first channel's input signal). FIG. 9(a) shows, for the case that $S_R$ are always turned off ($C_p$ are not discharged), that the first channel's output comprises fundamental component at 1 kHz, its 3rd harmonic component, and the output of the previous recorded channel, i.e., channel 16 (as a crosstalk component with -56 dB magnitude at 3.3 kHz). This crosstalk component was stored on $C_p$ and added to the output signal of the first channel 1 after $S_1$ is closed. This crosstalk can be minimized by the controlling of the reset switches $S_R$ according to the timing diagram in FIG. 8. The result is obtained in FIG. 9(b). The memory of channel 16 stored on $C_p$ is erased almost completely. The crosstalk component is thus reduced from -56 dB to -92 dB.

In summary of this disclosure, an amplifier circuit 20, a neuromodulation device 60, and a recording arrangement 80 are provided. The amplifier circuit 20 is configured to suppress a CM voltage signal in the input voltage signal 22, and consequently to reduce CMI, while not degrading the input impedance.

## Claims

1. An amplifier circuit (20) for amplifying a biological electrical signal (61), the amplifier circuit (20) comprising:

    an input stage (21) with two input terminals, configured to receive an input voltage signal (22), which is based on the biological electrical signal (61) and comprises a common mode, CM, voltage signal and a differential mode, DM, voltage signal;
    a differential transconductor (23) comprising a non-inverting input and an inverting input, each connected to one of the two input terminals, and comprising an output (24) for providing an output current signal based on the DM voltage signal;
    wherein the transconductor (23) is configured to provide a first CM voltage signal (25), which is tapped after the non-inverting input, and a second CM voltage signal (26), which is tapped after the inverting input; and
    a CM amplifier (27) configured to combine the first CM voltage signal (25) with the second CM voltage signal (26) to obtain a combined CM voltage signal, amplify the combined CM voltage signal with an inverting gain to obtain an inverted CM voltage signal (28), and provide the inverted CM voltage signal (28) to the non-inverting input and the inverting input of the transconductor (23).

2. The amplifier circuit (20) of claim 1, comprising:

a first capacitive feedback line (31) configured to feedback the inverted CM voltage signal (28) from the CM amplifier (27) to the non-inverting input of the transconductor (23); and
a second capacitive feedback line (32) configured to feedback the inverted CM voltage signal (28) from the CM amplifier (27) to the inverting input of the transconductor (23);
wherein each of the first and the second capacitive feedback line (31, 32) comprises a first capacitor ($C_{co}$).

3. The amplifier circuit (20) of claim 1 or 2, wherein:

each of the non-inverting input and the inverting input of the transconductor (23) is connected to one of the two input terminals of the input stage (21) via a respective second capacitor ($C_{in}$); and
the second capacitor ($C_{in}$) has a higher capacitance than the first capacitor (Cco).

4. The amplifier circuit (20) of one of the claims 1 to 3, wherein the transconductor (23) comprises:

a first transistor ($M_1$) connected with its gate to the non-inverting input and coupled with its source to a supply voltage ($V_{DD}$);
a second transistor ($M_2$) connected with its gate to the inverting input, connected with its source to the source of the first transistor ($M_1$), and coupled with its source to the supply voltage ($V_{DD}$);
a third transistor ($M_3$) connected with its gate to the non-inverting input, connected with its drain to the drain of the first transistor ($M_1$), and coupled with its source to a ground voltage (Vss); and
a fourth transistor ($M_4$) connected with its gate to the inverting input, connected with its drain to the drain of the second transistor ($M_2$), connected with its source to the source of the third transistor ($M_3$), and coupled with its source to the ground voltage (Vss);
wherein the first CM voltage signal (25) is tapped between the sources of the first and the second transistor ($M_1$, $M_2$), and the second CM voltage signal (26) is tapped between the sources of the third and the fourth transistor ($M_3$, $M_4$).

5. The amplifier circuit (20) of claim 4, configured such that a tail current of the source-connected third and fourth transistor ($M_3$, $M_4$) is higher than a tail current of the source-connected first and second transistor ($M_1$, $M_2$) in use of the amplifier circuit (20).

6. The amplifier circuit (20) of one of the claims 1 to 5, wherein the CM amplifier (27) comprises:

a summing junction (42) at which the first CM voltage signal (25) and the second CM voltage signal (26) are combined into the combined CM voltage signal;
wherein each of the first CM voltage signal (25) and the second CM voltage signal (26) is connected to the summing junction (42) via a respective third capacitor ($C_{ci}$).

7. The amplifier circuit (20) of claim 6, wherein:

the CM amplifier (27) further comprises a gain circuit (43) including a second transconductor (Ac), a fourth capacitor ($C_{cf}$) and a first resistor ($R_{cf}$); and
the gain circuit (43) is configured to amplify the combined CM voltage signal with the inverting gain to produce the inverted CM voltage signal (28).

8. The amplifier circuit (20) of one of the claims 1 to 7, further comprising:
a DM feedback circuit (33) configured to provide a feedback voltage signal (34), which is based on the output current signal of the transconductor (23), to the non-inverting input and the inverting input of the transconductor (23).

9. The amplifier circuit (20) of claim 8, wherein:

the DM feedback circuit (33) comprises a first capacitive feedback path and a second capacitive feedback path; and
each capacitive feedback path comprises a respective fifth capacitor ($C_f$) that is connected in parallel to a respective second resistor ($R_f$).

10. A neuromodulation device (60) for measuring one or more biological electrical signals (61) in response to a neural modulation of biological tissue, wherein the neuromodulation device (60) comprises one or more amplifier circuits (20) of one of the claims 1 to 9.

11. The neuromodulation device (60) of claim 10, further comprising:
an array of electrodes (71) comprising a first set of electrodes (72) and a second set of electrodes (73), wherein each electrode (72) of the first set is connected to a respective amplifier circuit (20) of the one or more amplifier circuits (20), and wherein each electrode (73) of the second set is configured to cause a respective neural modulation of the biological tissue.

12. The neuromodulation device (60) of claim 11, wherein each electrode (73) of the second set comprises a stimulator (62), which is configured to provide a stimulation signal (63) to cause the neural modulation of the biological tissue.

13. The neuromodulation device (60) of one of the claims 10 to 12, comprising:

multiple of the amplifier circuits (20);
wherein the amplifier circuits (20) share a single CM amplifier (27); and
the CM amplifier (27) is configured to provide, for each of the amplifier circuits (20), a respective inverted CM voltage signal (28) to the non-inverting input and the inverting input of the transconductor of that amplifier circuit (20).

14. The neuromodulation device (60) of claim 13, further comprising:

one or more shared buffers (81) connected to the multiple amplifier circuits (20) and to the single CM amplifier (27), wherein the output of each amplifier circuit (20) is connected by a respective switch (S1, S2, S3, $S_N$) to the shared buffers (81);
wherein the neuromodulation device (60) is configured to sequentially operate the switches (S1, S2, S3, $S_N$), which causes the multiple amplifier circuits (20) to sequentially output their respective current signal and causes the CM amplifier (27) to sequentially provide the respective inverted CM voltage signal (28) to the respective transconductor (23) of one of the amplifier circuits (20).

15. The neuromodulation device (60) of one of the claims 10 to 14, further comprising:

a bi-directional neural interface comprising at least one needle (74);
wherein the neural interface is configured to provide the neural stimulation to the biological tissue, to receive the one or more biological electrical signals (61), and to provide them to the one or more amplifier circuits (20).

FIG. 1

**FIG. 2**

**FIG. 3**

FIG. 4

**(a)**

**(b)**

**FIG. 5**

20

Feedback control

62

Stimulator

Artifacts

CSF

60

neural signal

61

63

**FIG. 6**

FIG. 7

FIG. 8

**(a)**

**(b)**

FIG. 9

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HONG SOONSEONG ET AL: "A Power-Efficient Low-Noise Neural Recording Amplifier IC with High Tolerance to Stimulation Artifacts", 2022 19TH INTERNATIONAL SOC DESIGN CONFERENCE (ISOCC), IEEE, 19 October 2022 (2022-10-19), pages 306-307, XP034289646, DOI: 10.1109/ISOCC56007.2022.10031576 [retrieved on 2023-02-07] | 1,2,8-15 | INV. H03F3/45 A61B5/305 |
| A | * page 306, left-hand column, line 12 – page 307, right-hand column, line 16; figures 1-5 * | 3-7 | |
| A | PÉREZ-PRIETO NORBERTO ET AL: "Recording Strategies for High Channel Count, Densely Spaced Microelectrode Arrays", FRONTIERS IN NEUROSCIENCE, vol. 15, 13 July 2021 (2021-07-13), pages 1-21, XP093128989, CH ISSN: 1662-453X, DOI: 10.3389/fnins.2021.681085 Retrieved from the Internet: URL:https://www.frontiersin.org/journals/neuroscience/articles/10.3389/fnins.2021.681085/full#B3> * page 1, line 5 – page 19, left-hand column, line 12; figures 1-13 * | 1-15 | |

TECHNICAL FIELDS SEARCHED (IPC)

H03F
A61B

-/--

The present search report has been drawn up for all claims

| Place of search | Date of completion of the search | Examiner |
|---|---|---|
| The Hague | 8 February 2024 | Fedi, Giulio |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

# EUROPEAN SEARCH REPORT

Application Number

EP 23 19 3810

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XU JIAWEI ET AL: "Active Electrodes for Wearable EEG Acquisition: Review and Electronics Design Methodology", IEEE REVIEWS IN BIOMEDICAL ENGINEERING, vol. 10, 29 December 2017 (2017-12-29), pages 187-198, XP011675427, ISSN: 1937-3333, DOI: 10.1109/RBME.2017.2656388 [retrieved on 2017-12-29] * page 187, left-hand column, line 22 – page 196, right-hand column, line 16; figures 1-27 * ----- | 1-15 | |
| A | US 5 392 784 A (GUDAITIS ALGIRD M [US]) 28 February 1995 (1995-02-28) * column 1, line 8 – column 6, line 62; figures 1-5 * ----- | 1-15 | |

TECHNICAL FIELDS
SEARCHED (IPC)

The present search report has been drawn up for all claims

| Place of search | Date of completion of the search | Examiner |
|---|---|---|
| The Hague | 8 February 2024 | Fedi, Giulio |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 2

**EP 4 518 155 A1**

## ANNEX TO THE EUROPEAN SEARCH REPORT
## ON EUROPEAN PATENT APPLICATION NO.

EP 23 19 3810

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2024

| Patent document cited in search report | Publication date | Patent family member(s) | Publication date |
|---|---|---|---|
| US 5392784 A | 28-02-1995 | DE 4417609 A1 | 23-02-1995 |
| | | JP H0794973 A | 07-04-1995 |
| | | US 5392784 A | 28-02-1995 |

EPO FORM P0459

For more details about this annex : see Official Journal of the European Patent Office, No. 12/82